# EUROPEAN PATENT SPECIFICATION

(11) **EP 1 165 056 B1**
(45) Date of publication and mention of the grant of the patent: **08.10.2003**
(21) Application number: 00925164.6
(22) Date of filing: 03.04.2000
(51) Int. Cl.: A61K 9/50, A61K 9/16

(54) **A PROCESS FOR THE MICROENCAPSULATION OF MEDICAMENTS**
VERFAHREN ZUR MIKROVERKAPSELUNG VON ARZNEISTOFFEN
PROCEDE DE MICROENCAPSULATION DE MEDICAMENTS

(30) Priority: 09.04.1999 IT MI990726
(43) Date of publication of application: 02.01.2002
(73) Proprietor: Pharmaplus S.r.l., 20013 Magenta (IT)
(72) Inventor: VALENTI, Fabrizio, I-20013 Magenta (IT)
(74) Representative: Minoja, Fabrizio, Dr.
(86) International application number: EP0002954
(87) International publication number: WO00061119

(56) References cited:
- WO-A-97/18798
- DE-A- 19 729 487
- DE-B- 1 207 374
- US-A- 3 082 154

## Description

The present invention relates to a microencapsulation for masking the unpleasant bitter taste of water-insoluble medicaments by means of one or more water-insoluble substances having melting point lower than the melting point of the medicament to be microencapsulated, and to the pharmaceutical compositions containing said microencapsulated medicaments.

The present invention relates to pharmaceutical preparations, more specifically tablets, chewable tablets, suspensions mainly in aqueous carrier and in general oral pharmaceutical forms containing a medicament in the form of microcapsules, the process for the preparation of microencapsulated medicaments, the pharmaceutical formulations prepared starting from said medicaments and the methods for the preparation thereof.

In the following, "medicament" means a therapeutically active, organic or inorganic compound, whereas "microencapsulated medicament" relates to a medicament particle or particles aggregates, of varying thickness, coated with one or more organic filmogenic agents, sparingly water-soluble and free from taste.

"Conventional pharmaceutical excipients" means all the excipients used for the formulation of pharmaceutical forms, such as ligands, suspending agents, sweetening agents, flavours, stabilizing agents, dyes, wetting agents and the like.

"Coating agent" means one or more organic, natural or synthetic, non toxic, free from taste, substances conventionally used for the preparation of pharmaceutical formulations.

Microencapsulation is a relatively recent technology which allows to coat particles of solids or small drops of liquids, in single form or as aggregates, with substances having polymeric structure thereby obtaining microcapsules having a size of about 0.1-1000 micron.

Microencapsulation techniques mainly used can be grouped in three classes:
- processes utilizing phase separation;
- processes based on interface polymerization;
- physical processes, such as centrifugation, extrusion and the like.

In practice, most microencapsulation procedures make use of the phase separation technique. Examples are disclosed in: EP 212,751, US 4,766,012, EP 413,533, US 4,460,563, US 4,462,982, US 4,835,187.

DE 1207374 discloses a process for the stabilisation of ascorbic acid by melt granulation with fatty substances. DE 19729487 discloses controlled-release formulations obtained by melt granulation.

US 3082154 discloses anti-malarial chloroquine formulations wherein the medicament is coated with fatty substances. WO97/18798 discloses a prompt-release pharmaceutical composition comprising microgranules coated with cetyl alcohol and stearyl alcohol or with t-butyl alcohol.

Physical processes are generally not very used in that they provide unsatisfactory microcapsules with hardly reproducible results.

The present invention makes use of a physical process combined with a highly efficient mechanical action capable of overcoming the drawbacks mentioned above.

The process of the invention is based on the capability of a hydrophobic substance, when heated above its melting point, of homogeneously distributing as a thin layer on medicament crystals or crystal aggregates kept in strong motion; the subsequent cooling causes the coating agent to solidify around the particle or particle aggregates, thus resulting in microcapsules free from taste and odour.

The present invention therefore provides a process for the microencapsulation of a medicament by means of at least one coating agent, which comprises the following steps:
a) Mixing the medicament in the form of crystals or crystal aggregates with the coating agent;
b) Heating the mixture, kept under strong stirring, to cause the only coating agent to melt;
c) Keeping the mixture under stirring at the coating agent melting temperature;
d) Cooling the mixture with constant stirring;
e) Recovering the microcapsules.

For the preparation of the microcapsules according to the present invention, the amount of coating for use compared with the medicament should range between 1 and 50%, preferably between 2.5 and 25%, more preferably between 5 and 20%.

The coating agents can be selected from water-insoluble substances, generally of lipid nature, pharmaceutically acceptable and with melting point lower than the medicament to be microencapsulated.

Examples of coating agents comprise:
**Long chain aliphatic alcohols such as**: cetyl alcohol m.p. 49°C, myristyl alcohol m.p. 38°C, stearyl alcohol m.p. 59.4-59.8°C.
**Long chain aliphatic acids:** such as stearic acid m.p. 69-70°C, palmitic acid m.p. 63-64°C, myristic acid m.p. 58.5°C, lauric acid m.p. 44-48°C, arachidic acid m.p. 75.5°C, etc.
**Esters of fatty acids and aliphatic alcohols:** methyl arachidate m.p. 47°C, ethyl arachidate m.p. 41°C, methyl stearate m.p. 38-39°C, ethyl stearate m.p. 33-35°C, glyceryl monostearate m.p. 56-58°C, glyceryl tripalmitate m.p. 66°C, glyceryl behenate (compritol), glyceryl palmitostearate (precirolate), lauroyl macrogol 32 glyceride (gelucire 44/14) stearoyl macrogol 32 glyceride (gelucire 50/13), castor oil.

The medicament can be mixed with the coating agent in horizontal or vertical working mixer granulators equipped with high shear mixing paddles, high efficiency crushers and heating and cooling jackets; this type of devices are commercially available under the trade marks Diosna, Collette, Zanchetta, Lödige.

The medicaments which can be used according to the invention should be insoluble both in the selected coating agent and in water, in case aqueous formulations have to be prepared. Moreover, the melting point of the medicaments should be markedly higher than the melting point of the coating agent.

Examples of medicaments which can conveniently be formulated according to the process of the invention comprise:
**nonsteroidal anti-inflammatory drugs:**
   ibuprofen, flurbiprofen, diclofenac, ketoprofen, naproxen, flufenamic acid, paracetamol, nimesulide, lornoxicam,
   indoprofen, indomethacin, ibufenac, etodolac, diflunisal, carbamazepine, bromfenac, aceclofenac, acetylsalicylic acid, codeine base, morphine base, tenoxicam, propyphenazon, ketorolac or the salts thereof;
   **antibiotics:** ampicillin, amoxicillin, cephalexin, erythromycin and derivatives, fosfomycin, miocamycin, rokitamycin,roxitromycin, or the salts thereof;
   **antitussives:** dextromethorphan hydrobromide, noscapine;
   **antihistamines:** oxatomide;
   **antiulcers:** cimetidine, famotidine, ranitidine, sucralfate;
   **mucolytics:**bromhexine.

Nonsteroidal antiinflammatories, in particular arylpropionic acids such as Ibuprofen, ketoprofen, naproxen, as well as antihistamines, such as oxatomide, are preferred.

Said active ingredients, particularly ibuprofen, encapsulated according to the invention, have substantially the same bioavailability as the non-microencapsulated drug.

The invention is described in greater detail in the following examples:

### Example 1

### Suspension containing Ibuprofen

Quali-quantitative composition for 100 ml of suspension:

| | |
|---|---|
| Ibuprofen | 2.000 g |
| Cetyl alcohol | 0.200 g |
| Saccharose | 50.000 g |
| Saccharose palmitostearate | 0.500 g |
| Methyl p-hydroxybenzoate | 0.100 g |
| Propyl p-hydroxybenzoate | 0.020 g |
| Dimethicone (10% emulsion) | 0.500 g |
| Aluminium magnesium silicate | 2.300 g |
| Caramel-toffee flavour | 0.200 g |
| Purified water | q.s. to 100.000 ml |

### Preparation procedure:

### - Preparation of the microcapsules

The necessary amount of Ibuprofen and cetyl alcohol is placed in a mixer granulator, mixing for about 10 minutes in the following conditions:
mixing paddle: 15-20 r.p.m.
crusher: 3000 r.p.m.

The stirred mass is heated by circulating hot water in the Diosna jacket, preventing the temperature of the product from exceeding 55°C. Once reached this temperature, stirring is continued for about 5 minutes, then cooling is started at a rate of about 1-2° per minute.

After reaching room temperature, the Ibuprofen microcapsules are unloaded and sieved through 0.5 mm, 0.25 mm and 0.10 mm sieves.

### - Preparation of the suspension

In a stainless steel container of suitable capacity, equipped with screw stirrer, almost all of the necessary water is loaded, and saccharose and preservatives are dissolved therein; solubilization has to be carried out at about 60-70°C. After cooling to room temperature (25°C), the syrup is added, in succession, with saccharose palmitostearate and the Ibuprofen microcapsules.

The whole is stirred to obtain a homogeneous suspension, then dimethicone, magnesium-aluminium silicate, flavour and the remaining amount of purified water are added. The suspension is kept under strong stirring for about 10 minutes, then it can be distributed in ampoules.

In vitro bioavailability tests were carried out with Ibuprofen microcapsules compared with non-microencapsulated Ibuprofen for evaluating the how the cetyl alcohol amount affects the active ingredient release rate.

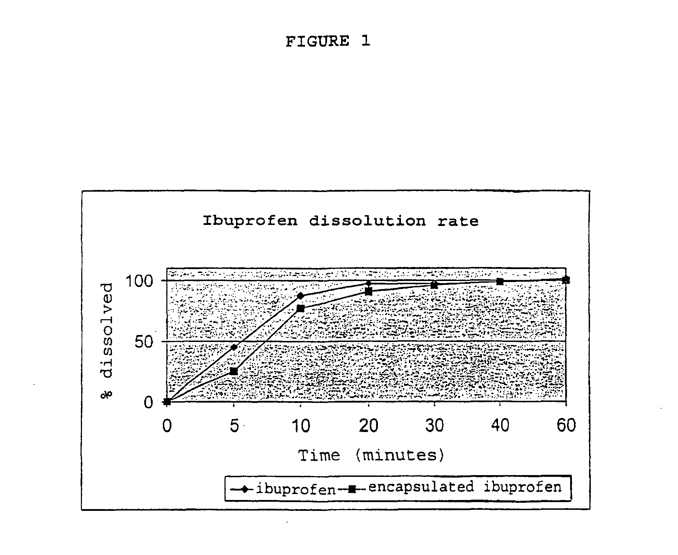

The procedure used is the same as reported in European Pharmacopoeia for Ibuprofen tablets;
Apparatus: type II
Dissolution medium: Phosphate Buffer pH = 7.2
Temperatures: 37°C ± 0.5°C
Rotation Speed: 100 rpm
The resulting diagram is reported in figure 1.

### Example 2

### Suspension containing acid diclofenac.

Quali-quantitative composition for 100 ml of suspension:

| | |
|---|---|
| Diclofenac acid | 0.500 g |
| Cetyl alcohol | 0.075 g |
| Saccharose | 50.000 g |
| Saccharose palmitostearate | 0.500 g |
| Sodium Benzoate | 0.500 g |
| Dimethicone (10% emulsion) | 0.500 g |
| Aluminium magnesium silicate | 2.300 g |
| Tropical Flavour | 0.200 g |
| Purified water | q.s. to 100.000 ml |

### Preparation procedure:

The same procedure as in example 1 is followed.

### Example 3

### Suspension containing on Naproxen

Quali-quantitative composition for 100 ml of suspension:

| | |
|---|---|
| Naproxen | 2.500 g |
| Compritol | 0.250 g |
| Saccharose | 50.000 g |
| Saccharose palmitostearate | 0.500 g |
| Sodium Benzoate | 0.500 g |
| Dimethicone (10% emulsion) | 0.500 g |
| Aluminium magnesium silicate | 2.300 g |
| Caramel flavour | 0.200 g |
| Purified water | q.s. to 100.000 ml |

### Preparation procedure:

The same procedure as in Example 1 is followed, the only changed parameter being the temperature in the microencapsulation step; in this case the temperature of the mass has to be raised to 85°C.

### Example 4

### Suspension containing Ketoprofen

Quali-quantitative composition for 100 ml of suspension:

| | |
|---|---|
| Ketoprofen | 0.500 g |
| Compritol | 0.050 g |
| Saccharose | 50.000 g |
| Saccharose palmitostearate | 0.500 g |
| Methyl p-hydroxybenzoate | 0.100 g |
| Propyl p-hydroxybenzoate | 0.020 g |
| Dimethicone (10% emulsion) | 0.500 g |
| Aluminium magnesium silicate | 2.300 g |
| Orange flavour | 0.200 g |
| Purified water | q.s. to 100.000 ml |

### Preparation procedure:

The same procedure as in Example 1 is followed, the only changed parameter being the temperature in the microencapsulation step; in this case the temperature of the mass has to be raised to 85°C.

### Example 5

### Chewable tablets containing Oxatomide

Quali-quantitative composition for a chewable tablet:

| | |
|---|---|
| Oxatomide | 0.015 g |
| Compritol | 0.002 g |
| Lactose | 0.263 g |
| Sorbitol | 0.400 g |
| Mannitol | 0.200 g |
| Aspartame | 0.050 g |
| Orange flavour | 0.060 g |
| Magnesium stearate | 0.010 g |

### Preparation procedure:

The same procedure as in Example 1 is followed.

Tablets are prepared according to the conventional water-humid granulation technology, the granulate being dried and mixed with flavours and sweetening agents, then pressed to the desired weight.

### Example 6

### Chewing-gum containing Oxatomide

Quali-quantitative unitary composition:

| | |
|---|---|
| Oxatomide | 7.500 mg |
| Compritol | 1.000 mg |
| Sorbitol | 0.800 g |
| Mannitol | 0.300 g |
| Isomalt | 0.060 g |
| Maltitol | 0.100 g |
| Mint flavour | 0.020 g |
| Glicamil | 0.025 g |
| Powder propolis | 0.025 g |
| Titanium dioxide | 0.005 g |
| Base gum | 0.320 g |

### Preparation procedure:

The same procedure as in Example 1 is followed.

The chewing-gum is prepared according to the conventional technology.

### Example 7

### Oral suspension containing 2%, 4%, 6% Ibuprofen.

Quali-quantitative composition for 100 ml of suspension:

| ***COMPONENTS*** | 2% Suspen. | 4% Suspen. | 6% Suspen. |
|---|---|---|---|
| Ibuprofen | 2.000 g | 4.000 g | 6.000 g |
| Cetyl alcohol | 0.223 g | 0.446 g | 0.569 g |
| Saccharose | 60.000 g | 60.000 g | 60.000 g |
| Saccharose | | | |
| palmitostearate | 0.500 g | 0.500 g | 0.500 g |
| Methyl p-hydroxy- | | | |
| benzoate | 0.100 g | 0.100 g | 0.100 g |
| Propyl p-hydroxy- | | | |
| benzoate | 0.020 g | 0.020 g | 0.020 g |
| EDTA sodium salt | 0.100 g | 0.100 g | 0.100 g |
| Citric acid | 0.200 g | 0.200 g | 0.200 g |
| Kaolin | 1.000 g | 1.000 g | 1.000 g |
| Dimethicone | 0.050 g | 0.050 g | 0.050 g |
| Xanthan gum | 0.500 g | 0.500 g | 0.600 g |
| Banana flavour | 0.150 g | 0.200 g | 0.300 g |
| Honey flavour | 0.050 g | 0.100 g | 0.150 g |
| Purified water | | | |
| q.s. to | 100 ml | 100 ml | 100 ml |

### Preparation procedure:

The same procedure as in Example 1 is followed.

Preparation of the suspension: as described in Example 1.

The in vivo bioavailability of the formulation containing 2% Ibuprofen was compared with that of a similar commercial formulation.

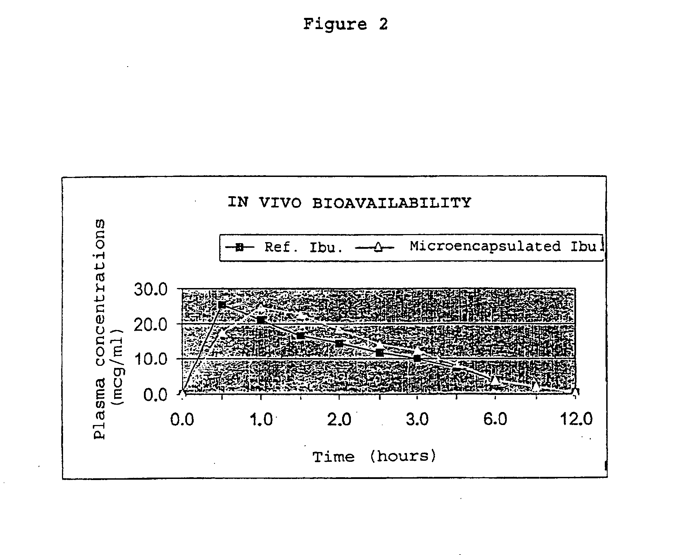

Annexed figure 2 shows the plasma concentrations obtained after administration of a dose of suspension equivalent to 200 mg Ibuprofen (reference Ibuprofen; microencapsulated Ibuprofen suspension).

The areas under each plasma concentration should be considered completely equivalent in that their ratio lies within the range 0.8-1.25 (calculated value 1.11).

The obtained results prove that the microencapsulation of Ibuprofen does not significantly affect bioavailability.

### Example 8

### Oral suspension containing 2%, 4%, 6% Naproxen.

Quali-quantitative composition for 100 ml of suspension

| ***COMPONENTS*** | 2%. Suspen | 4% Suspen. | 6% Suspen. |
|---|---|---|---|
| Naproxen | | | |
| (acid form) | 2.000 g | 4.000 g | 6.000 g |
| Cetyl alcohol | 0.223 g | 0.446 g | 0.669 g |
| Saccharose Saccharose | 60.000 g | 60.000 g | 60.000 g |
| palmitostearate | 0.500 g | 0.500 g | 0.500 g |
| Methyl p-hydroxy | | | |
| benzoate Propyl p-hydroxy- | 0.100 g | 0.100 g | 0.100 g |
| benzoate | 0.020 g | 0.020 g | 0.020 g |
| EDTA sodium salt | 0.100 g | 0.100 g | 0.100 g |
| Citric acid | 0.200 g | 0.200 g | 0.200 g |
| Kaolin | 1.000 g | 1.000 g | 1.000 g |
| Dimethicone | 0.050 g | 0.050 g | 0.050 g |
| Xanthan gum | 0.500 g | 0.500 g | 0.600 g |
| Banana flavour | 0.150 g | 0.200 g | 0.300 g |
| Honey flavour Purified water | 0.050 g | 0.100 g | 0.150 g |
| q.s. to | 100 ml | 100 ml | 100 ml |

### Preparation procedure:

The same procedure as in Example 1 is followed.

Preparation of the suspension: as described in Example 1.

### Example 9

### Oral suspension containing 0.5%, 1%, 5% Diclofenac

Quali-quantitative composition for 100 ml of suspension

| ***COMPONENTS*** | 0.5% Suspen. | 1% Suspen. | 5% Suspen. |
|---|---|---|---|
| Diclofenac | | | |
| (acid form) | 0.500 g | 1.000 g | 5.000 g |
| Cetyl alcohol | 0.075 g | 0.150 g | 0.750 g |
| Saccharose Saccharose | 60.000 g | 60.000 g | 60.000 g |
| palmitostearate Methyl p-hydroxy | 0.500 g | 0.500 g | 0.500 g |
| benzoate Propyl p-hydroxy- | 0.100 g | 0.100 g | 0.100 g |
| benzoate | 0.020 g | 0.020 g | 0.020 g |
| EDTA sodium salt | 0.100 g | 0.100 g | 0.100 g |
| Citric acid | 0.200 g | 0.200 g | 0.200 g |
| Kaolin | 1.000 g | 1.000 g | 1.000 g |
| Dimethicone | 0.050 g | 0.050 g | 0.050 g |
| Xanthan gum | 0.500 g | 0.500 g | 0.500 g |
| Banana flavour | 0.150 g | 0.200 g | 0.200 g |
| Honey flavour Purified water | 0.050 g | 0.100 g | 0.200 g |
| q.s. to | 100 ml | 100 ml | 100 ml |

### Preparation procedure:

The same procedure as in Example 1 is followed.

Preparation of the suspension: as described in example 1.

### Example 10

### Sugar-free oral suspension containing 2% Ibuprofen

Quali-quantitative composition for 100 ml of suspension:

| ***COMPONENTS*** | 0.5% Suspen. |
|---|---|
| Ibuprofen | 2.000 g |
| Cetyl alcohol | 0.223 g |
| Maltitol (70% sol.) | 60.000 g |
| Saccharose palmitostearate | 0.500 g |
| Domifen bromide | 0.010 g |
| Sodium saccharin | 0.100 g |
| EDTA sodium salt | 0.100 g |
| Citric acid | 0.200 g |
| Kaolin | 1.000 g |
| Dimethicone | 0.050 g |
| Xanthan gum | 0.500 g |
| Flavour | 0.300 g |
| Purified water q.s. to | 100 ml |

### Preparation procedure:

The same procedure as in Example 1 is followed.

Preparation of the suspension: as described in example 1.

## Claims

1. A process for the microencapsulation of a medicament selected from ibuprofen, ketoprofen, naproxen or oxatomide by means of at least one coating agent, which comprises the following steps:
a) Mixing the medicament in the form of crystals or crystal aggregates with the coating agent;
b) Heating the mixture, kept under strong stirring, to cause the only coating agent to melt;
c) keeping the mixture under stirring at the melting temperature of the coating agent;
d) Cooling the mixture with constant stirring;
e) Recovering the microcapsules.

2. A process as claimed in claim 1 wherein the coating agent is selected from long chain aliphatic alcohols, long chain aliphatic acids, esters of fatty acids with aliphatic alcohols.

3. Taste-masking Microcapsules obtained by the process of claims 1-2.

4. Oral pharmaceutical compositions containing the microcapsules of claim 3.

5. Oral pharmaceutical compositions according to claim 4 wherein the medicament is ibuprofen.

6. Pharmaceutical compositions as claimed in claim 4 or 5 in the form of tablets, chewable tablets, chewing-gums, suspensions.

7. A composition according to claim 5 in form of suspension having the following quali-quantitative composition for 100 ml:
| | |
|---|---|
| Ibuprofen | 2.000 g |
| Cetyl alcohol | 0.223 g |
| Maltitol (70% sol.) | 60.000 g |
| Saccharose palmitostearate | 0.500 g |
| Domifen bromide | 0.010 g |
| Sodium saccharin | 0.100 g |
| EDTA sodium salt | 0.100 g |
| Citric acid | 0.200 g |
| Kaolin | 1.000 g |
| Dimethicone | 0.050 g |
| Xanthan gum | 0.500 g |
| Flavour | 0.300 g |
| Purified water q.s. to | 100 ml |

8. Pharmaceutical compositions according to claim 5, in form of suspension having the following quali-quantitative composition for 100 ml of suspension:
| | |
|---|---|
| Ibuprofen | 2.000 g |
| Cetyl alcohol | 0.200 g |
| Saccharose | 50.000 g |
| Saccharose palmitostearate | 0.500 g |
| Methyl p-hydroxybenzoate | 0.100 g |
| Propyl p-hydroxybenzoate | 0.020 g |
| Dimethicone (10% emulsion) | 0.500 g |
| Aluminium magnesium silicate | 2.300 g |
| Caramel-toffee flavour | 0.200 g |
| Purified water | q.s. to 100.000 ml |

## Patentansprüche

1. Verfahren zur Mikroverkapselung eines Medikamentes, das aus Ibuprofen, Ketoprofen, Naproxen oder Oxatomid ausgewählt ist, mittels mindestens eines Beschichtungsmittels, welches die folgenden Schritte umfasst:
(a) Mischen des Medikamentes in Form von Kristallen oder Kristallaggregaten mit dem Beschichtungsmittel;
(b) Erhitzen der Mischung unter starkem Rühren, um das einzige Beschichtungsmittel veranlassen, zu schmelzen;
(c) Halten der Mischung unter Rühren bei der Schmelztemperatur des Beschichtungsmittels;
(d) Abkühlen der Mischung unter konstantem Rühren;
(e) Gewinnen der Mikrokapseln.

2. Verfahren nach Anspruch 1, wobei das Beschichtungsmittel ausgewählt ist aus langkettigen aliphatischen Alkoholen, langkettigen Fettsäuren, Estern von Fettsäuren und aliphatischen Alkoholen.

3. Geschmacksmaskierende Mikrokapseln, die durch das Verfahren nach den Ansprüchen 1-2 erhalten wurden.

4. Orale pharmazeutische Zusammensetzungen, die die Mikrokapseln nach Anspruch 3 enthalten.

5. Orale pharmazeutische Zusammensetzungen nach Anspruch 4, wobei das Medikament Ibuprofen ist.

6. Pharmazeutische Zusammensetzungen nach Anspruch 4 oder 5 in Form von Tabletten, Kautabletten, Kaugummis, Suspensionen.

7. Zusammensetzung nach Anspruch 5 in Form einer Suspension, die für 100 ml die folgende qualitativ-quantitative Zusammensetzung hat:
| | |
|---|---|
| Ibuprofen | 2,000 g |
| Cetylalkohol | 0,223 g |
| Maltitol (70%-ige Lsg.) | 60,000 g |
| Saccharosepalmitostearat | 0,500 g |
| Domifenbromid | 0,010 g |
| Natriumsaccharin | 0,100 g |
| EDTA-Natriumsalz | 0,100 g |
| Zitronensäure | 0,200 g |
| Kaolin | 1,000 g |
| Dimethicon | 0,050 g |
| Xanthangummi | 0,500 g |
| Aromastoff | 0,300 g |
| gereinigtes Wasser q.s. auf | 100 ml |

8. Pharmazeutische Zusammensetzungen nach Anspruch 5, in Form einer Suspension, die für 100-ml Suspension die folgende qualitativ-quantitative Zusammensetzung hat:
| | |
|---|---|
| Ibuprofen | 2,000 g |
| Cetylalkohol | 0,200 g |
| Saccharose | 50,000 g |
| Saccharosepalmitostearat | 0,500 g |
| Methyl-p-hydroxybenzoat | 0,100 g |
| Propyl-p-hydroxybenzoat | 0,020 g |
| Dimethicon (10%-ige Emulsion) | 0,500 g |
| Aluminiummagnesiumsilicat | 2,300 g |
| Karamel-Toffee-Aroma | 0,200 g |
| gereinigtes Wasser q.s. auf | 100,000 ml |

## Revendications

1. Procédé pour la microencapsulation d'un médicament choisi parmi l'ibuprofène, le kétoprofène, le naproxène ou l'oxatomide au moyen d'au moins un agent de revêtement, procédé qui comprend les étapes suivantes :
a) mélange du médicament sous forme de cristaux ou d'agrégats de cristaux avec l'agent de revêtement ;
b) chauffage du mélange, maintenu sous forte agitation, afin de faire fondre uniquement l'agent de revêtement ;
c) maintien du mélange sous agitation à la température de fusion de l'agent de revêtement ;
d) refroidissement du mélange sous agitation constante ;
e) récupération des microcapsules.

2. Procédé tel que revendiqué dans la revendication 1, dans lequel l'agent de revêtement est choisi parmi des alcools aliphatiques à longue chaîne, des acides aliphatiques à longue chaîne et des esters d'acides gras avec des alcools aliphatiques.

3. Microcapsules masquant le goût obtenues par le procédé des revendications 1-2.

4. Compositions pharmaceutiques pour administration par voie orale contenant les microcapsules de la revendication 3.

5. Compositions pharmaceutiques pour administration par voie orale selon la revendication 4 dans lesquelles le médicament est l'ibuprofène.

6. Compositions pharmaceutiques telles que revendiquées dans la revendication 4 ou 5 sous forme de comprimés, de comprimés que l'on peut mastiquer, de chewing-gums, de suspensions.

7. Composition selon la revendication 5 sous forme de suspension ayant la composition qualitative-quantitative suivante pour 100 ml :
| | |
|---|---|
| Ibuprofène | 2,000 g |
| Alcool cétylique | 0,223 g |
| Maltitol (sol. à 70%) | 60,000 g |
| Palmitostéarate de saccharose | 0,500 g |
| Bromure de domifène | 0,010 g |
| Saccharine sodique | 0,100 g |
| Sel de sodium de l'EDTA | 0,100 g |
| Acide citrique | 0,200 g |
| Kaolin | 1,000 g |
| Diméthicone | 0,050 g |
| Gomme de xanthane | 0,500 g |
| Arôme | 0,300 g |
| Eau purifiée q.s. pour | 100 ml |

8. Compositions pharmaceutiques selon la revendication 5, sous forme de suspension ayant la composition qualitative-quantitative suivante pour 100 ml de suspension :
| | |
|---|---|
| Ibuprofène | 2,000 g |
| Alcool cétylique | 0,200 g |
| Saccharose | 50,000 g |
| Palmitostéarate de saccharose | 0,500 g |
| p-Hydroxybenzoate de méthyle | 0,100 g |
| p-Hydroxybenzoate de propyle | 0,020 g |
| Diméthicone (émulsion à 10%) | 0,500 g |
| Silicate d'aluminium et de magnésium | 2,300 g |
| Arôme de caramel | 0,200 g |
| Eau purifiée q.s. pour | 100,000 ml |
